(19) Europäisches Patentamt
European Patent Office
Office européen des brevets

(11) Publication number: **0 106 926**
**A1**

(12) **EUROPEAN PATENT APPLICATION**

(21) Application number: **82305740.1**

(22) Date of filing: **28.10.82**

(51) Int. Cl.³: **C 07 D 233/94**, A 61 K 31/415

(43) Date of publication of application: **02.05.84**
**Bulletin 84/18**

(84) Designated Contracting States: **AT BE CH DE FR IT LI NL SE**

(71) Applicant: **DIOMED DEVELOPMENTS LIMITED, Tatmore Place, Gosmore Nr. Hitchin, Hertfordshire, SG4 7QR (GB)**

(72) Inventor: **Whitefield, Martin, 19 Parkway, London, NW11 (GB)**
Inventor: **Weinstock, Marcel, 8 Kings Drive, Edgware Middlesex (GB)**

(74) Representative: **Collier, Jeremy Austin Grey et al, J.A.Kemp & Co. 14, South Square Gray's Inn, London WC1R 5EU (GB)**

(54) **Nitroimidazole compounds and compositions useful in the treatment of acne.**

(57) Metronidazole retinoate and mizonidazole retinoate, with the formula (I)

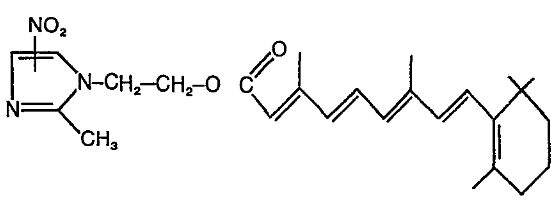

(I)

which are new compounds, are useful in the treatment of acne by topical application.

Pharmaceutical compositions comprising retinoic acid or a lower alkyl ester thereof and an ester of metronidazole or mizonidazole with an acid of formula RCOOH in which R is an aliphatic residue such that RCOOH is an aliphatic acid of 3 to 10 carbon atoms, are useful for the same purpose.

EP 0 106 926 A1

DESCRIPTION

"NITROIMIDAZOLE COMPOUNDS AND COMPOSITIONS USEFUL IN THE TREATMENT OF ACNE"

This invention relates to the compounds and compositions useful in the treatment of acne.

Acne is a disfiguring and frequently embarrassing condition which affects certain areas of the skin which are rich in sebaceous glands, especially the face, neck and back. It occurs most frequently in adolescents. While the precise cause of acne is not fully understood, it results from an abnormality of the sebaceous glands and blockage of the pilosebaceous ducts owing to a combination of factors. These include hyperkeratosis of the follicular duct, an increase in the amount and change in the composition of the sebum, and the colonisation of the sebaceous follicles by bacteria, especially Propionibacterium or Corynebacterium acnes. These effects produce the characteristic acne lesions, chiefly comedones, papules and pustules together with generalised inflammation. A variety of therapeutic treatments for acne are currently in use, and these are based in the main on keratolytics which reduce the hyperkeratinisation or on antibiotics which minimise the effects of the bacterial infection.

One method of treatment which has attracted

considerable interest in recent years is the use of retinoic acid (Vitamin A acid) applied topically to the lesions. Retinoic acid has a very marked keratolytic effect on the epidermis and a proportion of acne patients benefit from its use. It causes peeling of the skin and unblocks the pilosebaceous ducts. However, since retinoic acid is highly irritant, some patients do not benefit from its use and may indeed suffer undesirable side effects.

It has also been proposed to treat acne by oral antibiotic therapy using, in particular, tetracyclines. It is believed that where this treatment is successful, it is the action of the antibiotic on harmful bacterial metabolites which is responsible for the beneficial effect. Another method of treatment of some interest involves the topical application of ethyl lactate to the affected area. The bacterial esterases in the sebaceous ducts hydrolyse the ethyl lactate with liberation of lactic acid which suppresses the growth of the bacteria. Thus the bacterial are themselves responsible for producing the hydrolysis of the ethyl lactate which prevents their further growth.

The present invention provides novel compounds and compositions for use in the treatment of acne. The Propionibacterium acnes present in the sebaceous glands

in acne are predominantly anaerobic which explains why tetracyclines do not actually destroy them directly. Two agents which are known to be highly effective against anaerobic bacteria are the nitroimidazoles, metronidazole and mizonidazole. However, these compounds have poor solubility in lipids and cannot therefore be applied directly to skin with any expectation that they will reach the interior of the sebaceous glands populated by the bacteria. The present invention provides nitroimidazole derivatives and compositions which are effective in the treatment of acne.

The invention provides, as new compounds useful in the treatment of acne, the nitroimidazole derivatives of the formula:

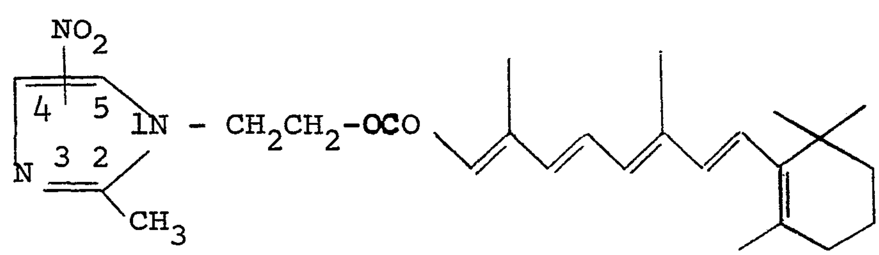

(I)

where the nitro group is in the 4- or 5-position of the imidazole ring. These compounds are the retinoates of metronidazole (the 5-nitro compound) and mizonidazole (the 4-nitro compound). These compounds are lipid-soluble and when applied topically to an

area of skin affected by acne, penetrate well into the epidermis. When the compounds reach the sebaceous glands, they are hydrolysed by the bacterial esterases present, thus liberating both the nitroimadazole itself which is then effective against those anaerobic bacteria, and retinoic acid, which exerts its beneficial keratolytic effect on the hyperkeratotic lining of the pilosebaceous duct and the keratin anchoring the comedones. Consequently, by esterifying the nitroimidazole derivative with retinoic acid, the beneficial effects of both are secured in the place, namely the interior of the pilosebaceous duct, where they are required.

Alternatively, according to a further feature of the invention, a similar effect can be secured by applying to the skin affected by acne a pharmaceutical composition comprising retinoic acid or a lower alkyl ester thereof and a nitroimidazole ester of the formula:

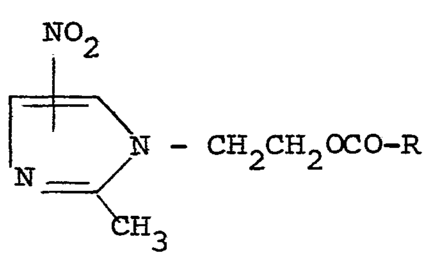

(II)

in which R is an aliphatic residue such that RCOOH is an aliphatic carboxylic acid of 3 to 10 carbon atoms. However R should be substantially free of groups which

would make the ester insoluble in lipids. Thus groups normally associated with water-solubility, e.g. hydroxyl and carboxyl, are not desirable. Conveniently R is a residue of an alkanecarboxylic acid of 3 to 10 carbon atoms, e.g. propionic acid, butyric acid or valeric acid.

When such a composition is applied to skin affected by acne, the retinoic acid penetrates to the pilosebaceous ducts with the nitroimidazole ester, and the latter is hydrolysed *in situ* by the esterases present. The desirable effect of retinoic acid and the nitroimidazole itself are thus secured in the affected areas. Of course, if a retinoic acid ester is used, such an ester is also hydrolysed in the vicinity of the sebaceous gland with liberation of free retinoic acid.

The aforesaid retinoate of nitroimidazole may be made by reaction of metronidazole or mizonidazole with retinoic acid chloride, prepared *in situ*, e.g. by the action of thionyl chloride on retinoic acid in an inert solvent in the presence of a base. For example, the nitroimidazole may be reacted with the acid chloride in solution in tetrahydrofuran, in the presence of an organic base soluble in the reaction mixture such as pyridine. This process is illustrated by the following Example.

EXAMPLE

Metronidazole Retinoate (2-Methyl-5-Nitroimidazole-1-Ethanol Retinoate)

To a solution of retinoic acid (5 g) and pyridine (1.6 g), in dry tetrahydrofuran (50 ml), thionyl chloride (2.1 g) in tetrahydrofuran (5 ml) was added at 0°C. over 15 minutes, under nitrogen, with stirring. The mixture was stirred at room temperature for 1 hour and then filtered. The filtrate was added over 10 minutes at 35°C. to a solution of 2-methyl-5-nitroimidazole-1-ethanol (3 g), 4-dimethylaminopyridine (0.1 g), and pyridine (1.5 g) in dry tetrahydrofuran (90ml). The mixture was stirred under reflux for 8 hours. A portion of the tetrahydrofuran (ca 60 ml) was then distilled off and the residue stirred under reflux for a further 8 hours. The reaction mixture was filtered and the solvent evaporated *in vacuo*. The residue was dissolved in chloroform (100 ml) and the solution washed with 2N sulphuric acid (2 x 50 ml), water (50 ml), 10% aqueous sodium bicarbonate (50 ml), and water (50 ml). The chloroform solution was then dried over sodium sulphate and evaporated *in vacuo*. The residue was purified by multi-funnel liquid-liquid extraction between light petroleum spirit (b.p. 40-60°C.) and 95% aqueous methanol to yield 2-methyl-5-nitroimidazole-1-ethanol retinoate as an orange-yellow gum (6 g) which solidifies on cooling.

The purity was shown by TLC (silica gel plates; ethyl acetate/light petroleum 1:1) to be >99%.

The N.M.R. spectrum showed peaks at 0.9 ppm (gem. dimethyl protons in acid component), 2.2 ppm ($CH_3$-C=C- protons in acid component), 6.1 ppm (vinylic protons in acid component), and 8 ppm (vinylic proton in imidazole ring of alcohol component). The N.M.R. data confirmed that the ester contained the essential characteristics of both the metronidazole and retinoic acid moieties.

Further identification of the ester was confirmed by hydrolysis. Under alkaline conditions retinoic acid was obtained as the only organic soluble product. Under acidic conditions metronidazole was obtained as the only identifiable product in the aqueous phase.

Esters of metronidazole and mizonidazole with aliphatic carboxylic acids of the formula RCOOH (in which R is as hereinbefore defined) may be prepared by well established procedures from metronidazole or mizonidazole and either a halide of the acid or the free acid itself.

The nitroimidazole retinoates of formula I may be applied to skin affected by acne in an appropriate pharmaceutical composition for topical application. In addition to the active ingredient, such compositions may contain appropriate inert diluents known for use in pharmaceutical compositions for topical application to the skin. The composition must, of course, contain sufficient

of the retinoate to produce the desired effect. A concentration of from 0.1 to 5.0% by weight of the composition is generally appropriate. In order to assure maximum stability of the composition, it may be formulated in a substantially anhydrous ointment base containing an appropriate oil-soluble antioxidant such as 2,6-ditertiary butyl para-cresol. Alternatively, it may be formulated as a cream, when the retinoate would be incorporated into the oleaginous phase while a water-soluble antioxidant such as ascorbic acid would be dissolved in the aqueous phase. In lower concentrations it may also be used as an alcoholic solution or gel. The aforesaid pharmaceutical compositions comprising retinoic acid or a lower alkyl ester thereof and a nitroimidazole ester of formula II may be similarly made up using conventional diluents compatible with the active ingredients also including alcoholic solutions or gels for lower concentrations. It is normally appropriate for such compositions to contain 0.01 to 5.0% by weight of the retinoic acid or ester thereof and 0.1 to 5.0% by weight of the nitroimidazole ester.

The following Examples of suitable formulations illustrate the invention.

| | Ingredients | Parts by weight |
|---|---|---|
| Example I | Metronidazole Retinoate | 0.5 |
| | Antioxidant | 0.2 |
| | White soft paraffin to | 100.0 |

| | Ingredients | Parts by weight |
|---|---|---|
| Example II | Metronidazole Retinoate | 0.5 |
| | White soft paraffin | 35.0 |
| | Cetomacrogol emulsifying wax | 10.0 |
| | Ascorbic acid | 0.2 |
| | Chlorocresol | 0.1 |
| | Purified water to | 100.0 |
| Example III | Metronidazole Propionate | 1.0 |
| | Retinoic acid | 0.025 |
| | Propylene glycol | 15.0 |
| | Alcohol to | 100.0 |
| Example IV | Metronidazole Propionate | 1.0 |
| | Ethyl retinoate | 0.5 |
| | Propylene glycol | 15.0 |
| | Alcohol to | 100.0 |

The above compositions are suitable for application to the skin to treat acne.

CLAIMS

1. A nitroimidazole derivative of the formula:

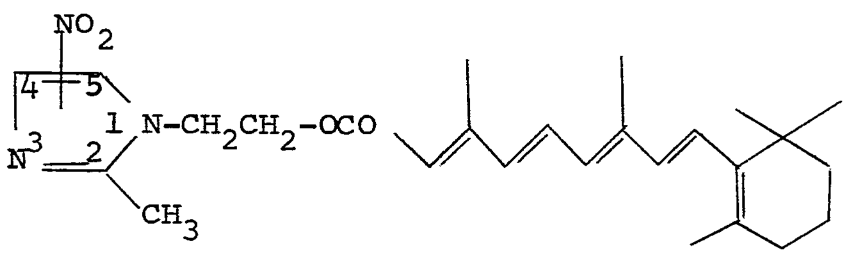

(I)

in which the nitro group is attached to the 4- or 5-position of the imidazole ring.

2. Metronidazole retinoate.

3. A pharmaceutical composition for topical application comprising retinoic acid or a lower alkyl ester thereof and a nitroimidazole ester of the formula:

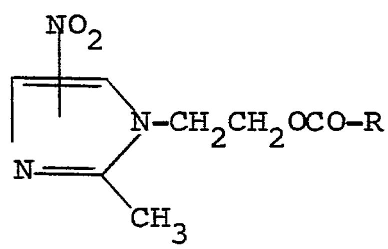

(II)

wherein R is an aliphatic residue such that RCOOH is an aliphatic carboxylic acid of 3 to 10 carbon atoms.

4. A pharmaceutical composition according to claim 3 comprising retinoic acid and the propionate, butyrate or valerate, of metronidazole.

5. A pharmaceutical composition according to claim 3 or 4, comprising 0.01 to 5.0% by weight of retinoic acid or a lower alkyl ester thereof and 0.1 to 5.0% by weight of a said nitroimidazole ester in

association with 90.0 to 99.89% by weight of a compatible pharmaceutical carrier.

6. Process for the preparation of a nitroimidazole retinoate as claimed in claim 1 which comprises reacting metronidazole or mizonidazole with a retinoic acid halide.

7. Process according to claim 6 in which the retinoic acid halide is produced *in situ* by the reaction of retinoic acid with thionyl chloride in the presence of a base.

8. Process according to claim 6 or 7, in which metronidazole is reacted with retinoic acid chloride in the presence of an inert organic solvent and an organic base.

European Patent Office

# EUROPEAN SEARCH REPORT

0106926

Application number
EP 82 30 5740

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | US - A - 3 803 165 (BEAMAN-TAUTZ) | | C 07 D 233/94<br>A 61 K 31/415 |
| A | GB - A - 901 060 (RHÔNE-POULENC) | | |
| A | FR - A - 1 393 963 (RHÔNE-POULENC)<br>-- | | |
| L | CHEMICAL ABSTRACTS, vol. 98, no. 13, 28-03-1983, pages 602-603, abstract no. 107.292x<br>COLUMBUS OHIO (US)<br>& GB - A - 2 097 783 (DERMAL LABORATORIES LTD.) (10-11-1982)<br>---- | | |
| | | | TECHNICAL FIELDS SEARCHED (Int. Cl. 3) |
| | | | C 07 D 233/00<br>A 61 K 31/00 |

~~The present search report has been drawn up for all claims~~

| Place of search | Date of completion of the search | Examiner |
|---|---|---|
| The Hague | 03-06-1983 | DE BUYSER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

## CLAIMS INCURRING FEES

The present European patent application comprised at the time of filing more than ten claims.

- [ ] All claims fees have been paid within the prescribed time limit. The present European search report has been drawn up for all claims.
- [ ] Only part of the claims fees have been paid within the prescribed time limit. The present European search report has been drawn up for the first ten claims and for those claims for which claims fees have been paid,

  namely claims:
- [ ] No claims fees have been paid within the prescribed time limit. The present European search report has been drawn up for the first ten claims.

## X LACK OF UNITY OF INVENTION

The Search Division considers that the present European patent application does not comply with the requirement of unity of invention and relates to several inventions or groups of inventions,
namely:

1. Claims 1-2,6-8: Nitroimidazole retinoates and preparation.

2. Claims 3-5: Pharmaceutical composition of a mixture of retinoic acid and a nitroimidazole ester.

- [ ] All further search fees have been paid within the fixed time limit. The present European search report has been drawn up for all claims.
- [ ] Only part of the further search fees have been paid within the fixed time limit. The present European search report has been drawn up for those parts of the European patent application which relate to the inventions in respect of which search fees have been paid,

  namely claims:
- [X] None of the further search fees has been paid within the fixed time limit. The present European search report has been drawn up for those parts of the European patent application which relate to the invention first mentioned in the claims,

  namely claims: 1-2,6-8